# EUROPÄISCHE PATENTSCHRIFT

(11) **EP 0 572 969 B1**
(45) Veröffentlichungstag und Bekanntmachung des Hinweises auf die Patenterteilung: **11.09.1996**
(21) Anmeldenummer: 93108776.1
(22) Anmeldetag: 01.06.1993
(51) Int. Cl.: A61M 13/00, A61M 15/00

(54) **Inhalator**
Inhalator
Inhalateur

(30) Priorität: 05.06.1992 DE 4218517
(43) Veröffentlichungstag der Anmeldung: 08.12.1993
(73) Patentinhaber: HOECHST AKTIENGESELLSCHAFT, 65926 Frankfurt am Main (DE)
(72) Erfinder: Schwab, Egon, W-6203 Hochheim am Main (DE); Loos, Hans-Joachim, W-6095 Ginsheim-Gustavsburg (DE); Ziegert, Günter, W-6230 Frankfurt am Main 80 (DE)

(56) Entgegenhaltungen:
- EP-A- 0 451 745
- WO-A-90/07351
- WO-A-91/06333

## Beschreibung

Die Erfindung betrifft einen Inhalator zum mehrfachen Inhalieren von pulverförmigen Arzneistoffen, der ein Gehäuse, eine Drehkappe, einen Deckel, einen Hohlzylinder, einen Zwischenboden, ein Dosierrad mit Dosierkammern und einen Ausblaskanal umfaßt, wobei das Gehäuse mit einer Vorratskammer versehen ist, deren Bodenteil eine Öffnung aufweist, deren Querschnitt dem der Dosierkammern entspricht, und der Ausblaskanal in einen Hohlkolben mündet, der den Ausblaskanal über eine Öffnung mit dem Innenraum des Zylinders verbindet.

Inhalationsgeräte der genannten Art sind aus der WO 91/06333 bekannt. Bei diesen Geräten wird ein atmosphärischer Überdruck dadurch erzeugt, daß man einen Kolben in einen Hohlzylinder schiebt. Die zusammengepreßte Luft wird durch eine Öffnung in dem Hohlzylinder über einen Hohlkolben in eine Vorratskammer entspannt, die mit einer pulverförmigen Substanz gefüllt und mit einem Ausblaskanal verbunden ist. Die Substanz wird dann plötzlich und vollständig durch den Ausblaskanal aus dem Inhalator abgegeben.

Nachteilig bei diesen Geräten ist ihr komplizierter mechanischer Aufbau sowie die Neigung zur Bildung von Konglomeraten infolge Luftfeuchtigkeit, die in ihr Kanalsystem eindringen kann. Hier will die Erfindung Abhilfe schaffen.

Die Erfindung löst die Aufgabe durch einen Inhalator mit den Merkmalen des Schutzanspruchs 1.

Die Verwirbelung der Arzneistoffe im Ausblaskanal kann noch durch radial verlaufende Bohrungen im Mundstück und eine Zerstäubereinrichtung im Ausblaskanal verbessert werden. Der Arzneistoffvorrat kann durch ein Sichtfenster in der Vorratskammer kontrolliert werden.

Neben praktischer Handhabung ermöglicht der Inhalator eine optimale Dosiergenauigkeit. Er kann durch einfaches Wechseln des Dosierrades den Dosiererfordernissen angepaßt werden. Er ist wartungsfrei und läßt sich leicht reinigen.

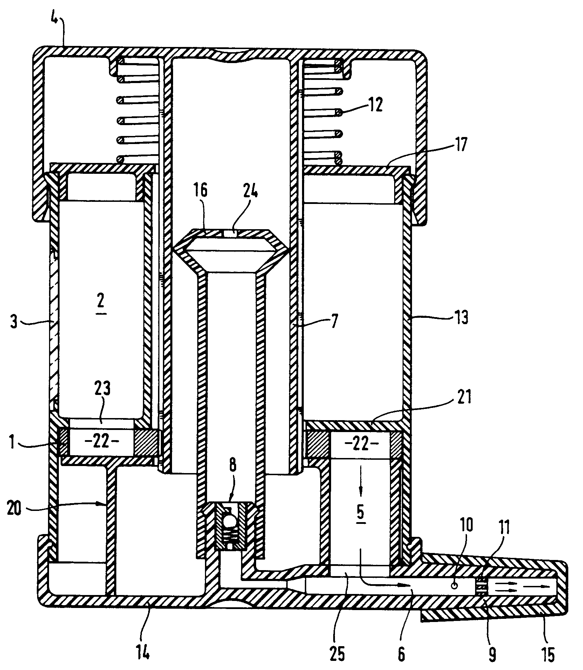

Ein Ausführungsbeispiel der Erfindung ist in der Figur dargestellt und wird im folgenden näher beschrieben:

Das Gehäuse (13) besitzt einen Zwischenboden (21) und ist mit einem Deckel (17) und einer Bodenplatte (14) verschlossen. Auf dem Deckel (17) stützt sich über eine Feder (12) eine Drehkappe (4) ab, deren Rand den oberen Rand der Gehäusewand umfaßt. Die Drehkappe (4) weist einen axial angeordneten Zylinder (7) auf, der durch den Deckel (17), den Zwischenboden (21) und eine Zarge (20) in das Gehäuse (13) ragt. Der Zylinder (7) ist gleichzeitig Antriebswelle für ein Dosierrad (1), das Dosierkammern (22) aufweist und das zwischen Zwischenboden (21) und Zarge (20) konzentrisch zum Zylinder (7) im Gehäuse (13) angeordnet ist. Das Gehäuse (13) weist eine Vorratskammer (2) für den Arzneistoff auf, der seinerseits in einer Kartusche enthalten sein kann. Die Vorratskammer ist mit einem Sichtfenster (3) versehen und weist eine Öffnung (23) auf, deren Querschnitt dem der Dosierkammer (22) entspricht. Die Bodenplatte (14) ist mit einem sich radial erstreckenden Mundstück (9) versehen, das einen Ausblaskanal (6) mit Ventil (8) enthält. Der Ausblaskanal (6) ist auf einer Seite mit einer Kappe (15) verschließbar und mündet mit dem anderen Ende in einen Hohlkolben (16), der sich von der Bodenplatte (14) ausgehend in den Zylinder (7) erstreckt. Über eine Öffnung (24) und Ventil (8) ist der Auslaßkanal (6) strömungsmäßig mit dem Innenraum des Zylinders (7) verbunden. Die Zarge (20) weist einen Schacht (5) auf, der die Dosierkammern (22) mit dem Ausblaskanal (6) über eine entsprechende Öffnung (25) im Mundstück (9) verbindet. Das Mundstück (9) kann mit radial verlaufenden Bohrungen (10) versehen sein, die in Ausblaskanal (6) münden. Ausblaskanal (6) kann eine Zerstäubereinrichtung (Siebplatte) (11) aufweisen. Der Arzneistoff fällt durch Öffnung (23) aus der Vorratskammer (2) in die Dosierkammer (22) des Dosierrades (1). Durch Drehen der Drehkappe (4) wird die dosierte Menge Pulver zum Austrittsschacht (5) transportiert und fällt durch diesen über Öffnung (25) in den Ausblaskanal (6). Die Applikation erfolgt durch Niederdrücken der Drehkappe (4) bis zum Anschlag, wodurch die im Zylinder (7) befindliche Luft bis zu einem definierten Druck komprimiert wird. Nach Erreichen des Druckes öffnet Ventil (8) und gibt die komprimierte Luft frei. Die Luft strömt zwangsweise in den Ausblaskanal (6), in welchem das dort deponierte Pulver vom Luftstrom aufgewirbelt und in Richtung des Mundstücks (9) geblasen wird. Im Mundstuck (9) befinden sich Seitenbohrungen (10), welche beim Inhalieren durch den Patienten im Ausblaskanal eine Injektorwirkung erzeugen. Pulverkonglomerate, wie sie bei feinen Pulvern vorkommen, werden durch den Luftstrom, der durch die Injektorwirkung verstärkt werden kann, gegen Siebplatte (11) geschleudert, dort zerschlagen und mikronisiert. Die Bodenplatte (14) mit Mundstück (9) ist abnehmbar, so daß letzteres bei Bedarf gereinigt werden kann. Um eine Doppeldosierung zu verhindern, ist der Inhalator mit einer Sperre (z. B. einer Kupplung, die bei Niederdrücken der Drehkappe ausrastet - nicht dargstellt) versehen, so daß erst wieder neu dosiert werden kann, wenn der Ausblasvorgang beendet ist. Die Drehkappe (4) wird nach dem Ausblasvorgang durch Feder (12) wieder in die Ausgangsposition gebracht.

## Patentansprüche

1. Inhalator zum mehrfachen Inhalieren von pulverförmigen Arzneistoffen, der ein Gehäuse (13), eine Drehkappe (4), einen Deckel (17), einen Hohlzylinder (7), einen Zwischenboden (21), ein Dosierrad (1) mit Dosierkammern (22) und einen Ausblaskanal (6) umfasst, wobei das Gehäuse (13) mit einer Vorratskammer (2) versehen ist, deren Bodenteil eine Öffnung (23) aufweist, deren Querschnitt dem der Dosierkammern (22) entspricht, und der Ausblaskanal (6) in einen Hohlkolben (16) mündet, der den Ausblaskanal (6) über eine Öffnung (24) mit dem Innenraum des Zylinders (7) verbindet,
**dadurch gekennzeichnet**, dass
a) das Gehäuse (13) eine Bodenplatte (14) und eine Zarge (20), die sich auf dieser Bodenplatte abstützt, aufweist und das Dosierrad (1) mit den Dosierkammern (22) zwischen dem Zwischenboden (21) und der Zarge (20) angeordnet ist,
b) die Drehkappe (4) durch eine Feder (12) auf dem Deckel (17) abgestützt ist, und der Zylinder (7) axial an dieser Drehkappe (4) angeordnet ist, durch den Deckel (17), den Zwischenboden (21) und die Zarge (20) in das Gehäuse (13) ragt und Antriebswelle für das Dosierrad (1) ist,
c) der Zwischenboden (21) das Bodenteil der Vorratskammer (2) bildet,
d) die Bodenplatte (14) mit einem in radialer Richtung angeordneten Mundstück (9) versehen ist, das den Ausblaskanal (6) aufweist,
e) der Ausblaskanal (6) mit einem Ventil (8) versehen ist,
f) der sich in den Zylinder (7) erstreckende Hohlkolben (16) an der Bodenplatte (14) befestigt ist,
g) die Zarge (20) einen Schacht (5) aufweist, der die Dosierkammern (22) mit dem Ausblaskanal (6) über eine Öffnung (25) im Mundstück (9) verbindet.

2. Inhalator nach Anspruch 1, dadurch gekennzeichnet, daß das Mundstück (9) mit radial verlaufenden Bohrungen (10) versehen und im Ausblaskanal (6) eine Zerstäubereinrichtung (11) angeordnet ist.

3. Inhalator nach Anspruch 1 oder 2, dadurch gekennzeichnet, daß die Vorratskammer (2) mit einem Sichtfenster (3) versehen ist.

## Claims

1. An inhaler for the repeated inhalation of pulverulent pharmaceuticals, which inhaler comprises a housing (13), a rotatable cap (4), a lid (17), a hollow cylinder (7), an intermediate floor (21), a dosing wheel (1) with dosing chambers (22) and a blow-out channel (6), with the housing (13) being provided with a supply chamber (2) whose floor part has an opening (23) whose cross-section corresponds to that of the dosing chambers (22), and the blow-out channel (6) opening out into a hollow piston (16) which connects the blow-out channel (6) by way of an opening (24) to the inner space of the cylinder (7),
**wherein**
a) the housing (13) has a base plate (14) and a frame (20) which is supported on this base plate, and the dosing wheel (1) is arranged together with the dosing chambers (22) between the intermediate floor (21) and the frame (20),
b) the rotatable cap (4) is supported on the lid (17) by a spring (12), and the cylinder (7) is arranged axially on this rotatable cap (4), projects through the lid (17), the intermediate floor (21) and the frame (20) into the housing (13) and is the drive shaft for the dosing wheel (1),
c) the intermediate floor (21) forms the floor part of the supply chamber (2),
d) the base plate (14) is provided with a mouthpiece (9) which is arranged in a radial direction, and which has the blow-out channel (6),
e) the blow-out channel (6) is provided with a valve (8),
f) the hollow piston (16) which extends into the cylinder (7) is fixed to the base plate (14),
g) the frame (20) has a shaft (5) which connects the dosing chambers (22) with the blow-out channel (6) by way of an opening (25) in the mouthpiece (9).

2. The inhaler as claimed in claim 1, wherein the mouthpiece (9) is provided with drilled holes (10) which extend radially, and a nebulizing device (11) is arranged in the blow-out channel (6).

3. The inhaler as claimed in claim 1 or 2, wherein the supply chamber (2) is provided with a viewing window (3).

## Revendications

1. Inhalateur pour inhaler plusieurs fois des substances médicinales sous forme de poudre, qui comprend un boîtier (13), un capuchon rotatif (4), un couvercle (17), un cylindre creux (7), un fond intermédiaire (21), une roue de dosage (1) comportant des chambres de dosage (22) et un conduit d'évacuation (6), le boîtier (13) étant pourvu d'une chambre de réserve (2) dont la partie de fond présente une ouverture (23) dont la section correspond à celle des chambres de dosage (22), et le conduit d'évacuation (6) débouche dans un piston creux (16) qui relie le conduit d'évacuation (6) à l'espace intérieur du cylindre (7) par une ouverture (24),
caractérisé en ce que
a) le boîtier (13) présente une plaque de fond (14) et un châssis (20) qui s'appuie sur cette plaque de fond, et la roue de dosage (1) est agencée avec les chambres de dosage (22) entre le fond intermédiaire (21) et le châssis (20),
b) le capuchon rotatif (4) s'appuie par l'intermédiaire d'un ressort (12) sur le couvercle (17), et le cylindre (7) est agencé axialement à ce capuchon rotatif, s'élève dans le boîtier (13) par le couvercle (17), le fond intermédiaire (21) et le châssis (20) et est un arbre moteur pour la roue de dosage (1),
c) le fond intermédiaire (21) forme la partie de fond de la chambre de réserve (2),
d) la plaque de fond (14) est pourvue d'une embouchure (9), agencée dans une direction radiale, qui comprend le conduit d'évacuation (6),
e) le conduit d'évacuation (6) est pourvu d'une soupape (8),
f) le piston creux (16), qui s'étend dans le cylindre (7), est fixé à la plaque de fond (14)
g) le châssis (20) présente une cavité (5) qui relie les chambres de dosage (22) au conduit d'évacuation (6) par une ouverture (25) dans l'embouchure (9).

2. Inhalateur selon la revendication 1, caractérisé en ce que l'embouchure (9) est pourvue de trous (10) s'étendant radialement et qu'un dispositif pulvérisateur (11) est agencé dans le conduit d'évacuation (6).

3. Inhalateur selon la revendication 1 ou 2, caractérisé en ce que la chambre de réserve (2) est pourvue d'une lucarne (3).
